# EUROPÄISCHE PATENTANMELDUNG

(11) **EP 1 252 884 A2**
(43) Veröffentlichungstag der Anmeldung: **30.10.2002**
(21) Anmeldenummer: 02009003.1
(22) Anmeldetag: 23.04.2002
(51) Int. Cl.: A61K 9/08

(54) **Orales Mittel für die Human- und Veterinärmedizin**

(30) Priorität: 26.04.2001 DE 10120428
(71) Anmelder: Gieselmann, Thomas, 56288 Kastellaun (DE)
(72) Erfinder: Gieselmann, Thomas, 56288 Kastellaun (DE)
(74) Vertreter: Müller-Gerbes, Margot, Dipl.-Ing.

(57) **Zusammenfassung**

Orales Mittel zur Verbesserung der Darmmotorik und Defäkation, als Diätetikum und/oder als Trägerlösung für Allophathika, Phytopharmaka, Homöopathika, Mineralstoffen, Enzymen, Vitaminen, Spurenelemente, Pharmaka oder Diagnostika, enthaltend auf ein Liter Wasser die folgenden Komponenten:
A) 0,5 bis 80 g mindestens eines Verdickungsmittels
C) 4 bis 100 g eines vernetzten unlöslichen Polyvinylpolyprrolidon (PVPP)
C) 0 bis 40 g Mannit,
D) 0 bis 20 g Aromastoffe
E) 0 bis 50 g eines Lösungsmittels für eine der Komponenten A,
F) 0 bis 5 g nichtionisches Tensid.

## Beschreibung

Die Erfindung betrifft ein orales Mittel für die Human- und Veterinärmedizin.

Als orale Mittel werden beispielsweise orale Trägerlösungen benötigt.

Eine orale Trägerlösung hat die folgenden Anforderungen zu erfüllen:
- sie darf nicht toxisch sein
- sie darf die Darmperistaltik nicht beeinflussen
- sie darf nicht resorbiert werden.

Des weiteren muss die Trägerlösung innigen Kontakt zum Endothel herstellen, den Darm homogen ausfüllen und den Darm - bei entsprechender Trinkmenge - dehnen.

Eine allgemein verwendbare Trägerlösung für pharmakologisch wirksame Stoffe und Diätetika wurde bisher nicht beschrieben.

Der Erfindung liegt die Aufgabe zugrunde, eine orale Multifunktions-Trägerlösung für die Human- und Veterinärmedizin für die Indikationen :
- Neigung zur Verstopfung
- funktionelle Darmträgheit
- Appetithemmung
- Substitution von Mineralstoffen, Vitaminen und Spurenelementen
- Zufuhr von Pharmaka vorzuschlagen.

Überraschenderweise wurde gefunden, dass eine Lösung gemäß der Zusammensetzung, enthaltend auf ein Liter Wasser die folgenden Komponenten
A) 0,5 bis 80 g mindestens eines Verdickungsmittels
B) 4 bis 100 g eines vernetzten unlöslichen Polyvinylpolypyrrolidon (PVPP),
C) 0 bis 40 g Mannit,
D) 0 bis 20 g Aromastoffe
E) 0 bis 50 g eines Lösungsmittels für eine der Komponenten A,
F) 0 bis 5 g nichtionisches Tensid.
diese Anforderung erfüllt. Eine Zusammensetzung dieser Art ist als orales Echokontrastmittel aus der DE 44 24 233 C1 bekannt. Es wurde nunmehr überraschend gefunden, daß diese Zusammensetzung auch bei Anwendung ein abnehmendes Hungergefühl, eine Regulierung der Stuhlfrequenz und abnehmende Beschwerden bei der Defäkation bewirkt. Diese Lösung eignet sich also in direktem Einsatz zur Verbesserung der Darmmotorik und Defäkation.

Die erfindungsgemäße Zusammensetzung erfüllt überraschenderweise die eingangs aufgeführten Aufgaben als orale Mulitfunktionsträgerlösung.

Wurden dieser Lösung Phytopharmaka, wie z.B. Madar, hinzugefügt, wurde in Einzelfällen eine schonende Gewichtsabnahme übergewichtiger Personen beobachtet.

In ausreichend hoher Dosierung ist die Lösung gemäß Patentanspruch somit als Laxans und Appetitbremse einzusetzen.

Insbesondere wurde überraschenderweise mittels Ultraschall nachgewiesen, dass die Darreichungsform sehr intensiv und langanhaltend an der Darmmucosa haftet und somit eine ideale Möglichkeit darstellt, Pharmaka und andere Stoffe, deren Resorption erwünscht ist, in dieser Lösung zu verpacken und oral zu applizieren. Die erfindungsgemäße Lösung gemäß Patentanspruch 1 ist somit auch in Verbindung mit Allopathika, Phytopharmaka, Homöopathika, Mineralstoffen, Spurenelemente, Vitaminen, auch als Diätetikum, als orales Mittel in Form einer oralen Trägerlösung verwendbar.

Alle für die erfindungsgemäße orale Trägerlösung eingesetzten Komponenten sind unter dem Gesichtspunkt auszusuchen, dass sie nicht toxisch sind, die Darmperistaltik nicht beeinflussen, nicht resorbiert werden, den Darm homogen ausfüllen und die Darmwände vollständig benetzen.

Als Verdickungsmittel kommen insbesondere organische natürliche Verdickungsmittel wie z.B. Guar-Gum, Xanthan, Pektin, Gelatine oder Alginate oder Agar-Agar, Tragant, Carrageen, Gummiarabicum, Polyosen, Stärke, Dextrine und/oder Casein und/oder organische abgewandelte Naturstoffe und/oder vollsynthetische organische Stoffe wie z. B. substituierte Zellulosen, wie Methylcellulose, Methylpropylcellulose, Hydroxipropylcellulose, Hydroxipropylmethylcellulose, Ethylcellulose, Polyvinylalkohole oder leicht in Wasser lösliche Polyvinylpyrrolidone (Povidone) in Frage.

Bevorzugt sind gut wasserlösliche organische natürliche Verdickungsmittel und auf Basis organisch abgewandelter Naturstoffe erhaltene Verdickungsmittel

Um die Darm-Mukosa besser zu benetzen, kann der Trägerlösung ein geeignetes pharmazeutisch bzw. lebensmittelrechtlich zugelassenes Tensid, bevorzugt ein nichtionisches Tensid zugesetzt werden. Hierfür kommt beispielsweise ein als nichtionisches Tensid wirksames Poloxamer in Frage. Es ist auch möglich, die sogenannten Zuckertenside einzusetzen. Auch andere lebensmittelrechtlich zugelassenen Tenside können für die gewünschten Zwecke benutzt werden.

Der erfindungsgemäßen Trägerlösung kann des weiteren Mannit zugesetzt werden, das neben der Süßstoffwirkung auch eine leicht abführende Wirkung aufweist. Eine solche abführende Wirkung ist auch für das Wohlbefinden des mit der erfindungsgemäßen Trägerlösung behandelten Patienten ebenfalls wünschenswert.

Der erfindungsgemäßen Trägerlösung können des weiteren Geschmackskorrigentien und eventuell auch ein Konservierungsmittel zugegeben werden. Hierfür kommen ebenfalls die lebensmittelrechtlich zugelassenen Aromastoffe und Konservierungsmittel in Frage.

Die Erfindung beruht insbesondere auf der Kombination aus mindestens einem Verdickungsmittel und einem quervernetzten unlöslichen Polyvinylpolypyrrolidon, das auch als Crospovidon bezeichnet wird, das zu dem gewünschten Erfolg führt.

Die bevorzugten Mengen der einzelnen Komponenten und zwar in der auf ein Liter Wasser berechneten Menge, womit eine durch den Patienten gut einnehmbare pastöse Lösung erhalten wird, sind:

### Komponenten:

A) 1 bis 40 g mindestens eines Verdickungsmittels,
B) 10 bis 60 g Polyvinylpolypyrrolidon (PVPP),
C) 8 bis 20 g Mannit,
D) 0,5 bis 1,5 g Aromastoffe,
E) 10 bis 30 g eines Lösungsmittels für eine der Komponenten A,
F) 0,1 bis 3 g nichtionisches Tensid.

Der erfindungsgemäßen Trägerlösung können des weiteren Phytopharmaka, wie Madar, Homöopathika, Phytotherapeutika Allopathika, wie Antibiotika, Sulfonamide, ASS, Wismut, Corticoide etc., Vitamine, Spurenelemente, Mineralstoffe und Kombinationen daraus hinzugefügt werden .

Um eine gleichmäßige Verteilung der einzelnen Komponenten in der Trägerlösung zu erhalten, kann es zweckmäßig sein, eines der Verdickungsmittel separat in einem Lösungsmittel zu verteilen und dann in dieser Vorverteilung dem Wasser zuzugeben.

Als ein solches zusätzliches Lösungsmittel (Komponente E) kommt beispielsweise Propylenglykol in geringen Mengen zum Einsatz. Auch andere geeignete und lebensmittelrechtlich zugelassene Lösungsmittel können hier eingesetzt werden. Eine bevorzugte Zusammensetzung einer erfindungsgemäßen Trägerlösung enthält auf einen Liter Wasser als Komponenten:
A) 2 bis 10 g Xanthan und 2 bis 3 g Povidone,
B) 20 bis 60 g PVPP,
C) 5 bis 30 g Mannit,
D) 0,1 bis 1,2 g Aromastoffe,
E) 10 bis 30 g Propylenglykol und
G) 0 bis 5 g Poloxamer.

Humanversuche mit gemäß der Erfindung zusammengesetzter Trägerlösung haben die gute Verträglichkeit und ihre überraschenden Eigenschaften erwiesen. Orale Trägerlösungen gemäß der Erfindung sind in Verbindung mit Diätetika und pharmakologisch wirksamen Stoffen besonders verträglich und wirkungssteigernd, insbesondere bei Indikationen wie Verstopfung, funktionelle Darmträgheit, Appetithemmung, Substitution von Mineralstoffen, Vitaminen und Spurenelementen und Zufuhr von Pharmaka.

Die Erfindung wird nachfolgend anhand zweier beispielhafter Zusammensetzungen einer Trägerlösung dargestellt.

### Beispiel 1

- **Komponente A:**: Xanthan-Gurn (Handelsbezeichnung Keltrol F der Firma Kelco) 4g Povidone (Handelsbezeichnung Kollidon 17 der Firma BASF) 20 g
- **Komponente B:**: PVPP (Handelsbezeichnung Polyplasdone X L der Firma GAF) 36 g
- **Komponente C:**: Mannit 10 g
- **Komponente D:**: Aromastoff 1.0 g
- **Komponente E:**: Propylenglykol 20 g
- **Wasser:**: 1 Liter (1000 g)

Zur Herstellung der Trägerlösung wurde das Mannit, das Povidon und der Aromastoff in dem Wasser gelöst. Danach wurde das PVPP suspendiert.
In einem separaten Gefäß wird das Xanthan in Propylenglykol homogen verteilt und unter Rühren der vorgenannten wässrigen Flüssigkeiten zugegeben.
Nach dem Quellen des Xanthan ist die Zubereitung gebrauchsfertig als Multifunktions-Trägerlösung.

Die Zugabe der Indikationsstoffe z.B. Allopathika, hat individuell gemäß galenischer Entwicklung zu erfolgen.

### Beispiel 2

- **Komponente A:**: Xanthan-Gurn (Handelsbezeichnung Keltrol F der Firma Kelco) 3g Povidone (Handelsbezeichnung Kollidon 17 der Firma BASF) 3 g
- **Komponente B:**: PVPP (Handelsbezeichnung Polyplasdone X L der Firma GAF) 40 g
- **Komponente C:**: Mannit 15 g
- **Komponente D:**: Aromastoff 1.0 g
- **Komponente E:**: Propylenglykol 20 g
- **Komponente F:**: Poloxamer 188 2.0 g
- **Wasser:**: 1 Liter (1000 g).

Zur Herstellung der Trägerlösung erfolgt analog wie in Beispiel 1 beschrieben. Die Komponente F wird zusammen mit dem Mannit und dem Povidon und den Aromastoffen in dem Wasser gelöst.

Die Zugabe der Indikationsstoffe z. B. Madar hat individuell gemäß galenischer Entwicklung zu erfolgen.

Die erfindungsgemäße Lösung kann zur Verbesserung der Darmmotorik und Defäkation, zur Hilfe bei Gewichtsabnahme, zur Vorbereitung von Ultraschalluntersuchungen im abdominellen Bereich, eingesetzt werden.

Das erfindungsgemäße orale Mittel ist geeignet zur Verwendung als Trägerlösung für diätetische oder pharmakologisch wirksame Stoffe, als Trägerlösung für Vitamine, Enzyme usw. als diätetisch wirksame Stoffe, als Trägerlösung für Vitamine, Steroide, Phyto, Allophatika, H1, H2, ATP-ase Hemmer etc., als pharmakologisch wirksame Stoffe oder auch als Trägerlösung für Perfluoro-Octylbromid, superparamagnetische Teilchen usw., als diagnostisch wirksame Stoffe.

## Patentansprüche

1. Orales Mittel zur Verbesserung der Darmmotorik und Defäkation, als Diätetikum und/oder als Trägerlösung für Allophathika, Phytopharmaka, Homöopathika, Mineralstoffen, Enzymen, Vitaminen, Spurenelemente, Pharmaka oder Diagnostika, enthaltend auf ein Liter Wasser die folgenden Komponenten:
A) 0,5 bis 80 g mindestens eines Verdickungsmittels
B) 4 bis 100 g eines vernetzten unlöslichen Polyvinylpolypyrrolidon (PVPP),
C) 0 bis 40 g Mannit,
D) 0 bis 20 g Aromastoffe
E) 0 bis 50 g eines Lösungsmittels für eine der Komponenten A,
F) 0 bis 5 g nichtionisches Tensid.

2. Orales Mittel nach Anspruch 1, **dadurch gekennzeichnet, daß** das organische natürliche Verdickungsmittel und/oder organische abgewandelte Naturstoff und/oder vollsynthetische organische Stoffe als Verdickungsmittel eingesetzt sind.

3. Orales Mittel nach Anspruch 1 oder 2, **dadurch gekennzeichnet, daß** als organische natürliche Verdickungsmittel Agar-Agar, Tragant, Carrageen, Alginate, Gummi arabicum, Pektine, Polyosen, Guar-Mehl, Stärke, Xanthan, Dextrine, Gelatine und/oder Casein eingesetzt sind.

4. Orales Mittel nach einem der Ansprüche 1 bis 3, **dadurch gekennzeichnet, daß** als Verdickungsmittel auf Basis organisch abgewandelter Naturstoffe Carboxymethylcellulose und/oder Celluloseether eingesetzt sind.

5. Orales Mittel nach einem der Ansprüche 1 bis 4, **dadurch gekennzeichnet, daß** als vollsynthetisches Verdickungsmittel mitteloder hochmolekulare Povidone (Polyvinylpyrrolidone) eingesetzt sind.

6. Orales Mittel nach einem der Ansprüche 1 bis 5, **dadurch gekennzeichnet, daß** mindestens eines der eingesetzten Verdickungsmittel gut in Wasser löslich ist.

7. Orales Mittel nach einem der Ansprüche 1 bis 6, **dadurch gekennzeichnet, daß** als Komponente F eine lebensmittelrechtlich zugelassenes als nichtionisches Tensid wirksames Poloxamer eingesetzt ist.

8. Orales Mittel nach einem der Ansprüche 1 bis 7, **dadurch gekennzeichnet, daß** als Lösungsmittel Propylenglykol eingesetzt ist.

9. Orales Mittel nach einem der Ansprüche 1 bis 8, enthaltend auf einen Liter Wasser die folgenden Komponenten:
A) 1 bis 40 g mindestens eines Verdickungsmittels,
B) 10 bis 60 g Polyvinylpolypyrrolidon (PVPP),
C) 8 bis 20 g Mannit,
D) 0,5 bis 1,5 g Aromastoffe,
E) 10 bis 30 g eines Lösungsmittels für eine der Komponenten A,
F) 0,1 bis 3 g nichtionisches Tensid.

10. Echokontrastmittel nach einem der Ansprüche 1 bis 9, **gekennzeichnet durch** eine Zusammensetzung, enthaltend als Komponenten:
A) 2 bis 10 g Xanthan und 2 bis 3 g Povidone,
B) 20 bis 60 g PVPP,
C) 5 bis 30 g Mannit,
D) 0,1 bis 1,2 g Aromastoffe,
E) 10 bis 30 g Propylenglykol und
F) 0 bis 5 g Poloxamer
